**Europäisches Patentamt**

**European Patent Office**

**Office européen des brevets**

(11) Veröffentlichungsnummer : **0 101 910
B1**

(12) **EUROPÄISCHE PATENTSCHRIFT**

(45) Veröffentlichungstag der Patentschrift :
**03.04.85**

(21) Anmeldenummer : **83107214.5**

(22) Anmeldetag : **22.07.83**

(51) Int. Cl.⁴ : **C 07 C 67/48, C 07 C 67/54,
C 07 C 67/60, C 07 C 69/44**

(54) Verfahren zur Reinigung von Aldehyde, Acetale und/oder ungesättigte Verbindungen enthaltenden Carbonsäureestern.

(30) Priorität : **30.07.82 DE 3228500**

(43) Veröffentlichungstag der Anmeldung :
**07.03.84 Patentblatt 84/10**

(45) Bekanntmachung des Hinweises auf die Patenterteilung : **03.04.85 Patentblatt 85/14**

(84) Benannte Vertragsstaaten :
**BE DE FR GB IT NL**

(56) Entgegenhaltungen :
**EP-A- 0 030 330
US-A- 4 189 599**

(73) Patentinhaber : **BASF Aktiengesellschaft
Carl-Bosch-Strasse 38
D-6700 Ludwigshafen (DE)**

(72) Erfinder : **Kummer, Rudolf, Dr.
Kreuzstrasse 6
D-6710 Frankenthal (DE)**
Erfinder : **Schneider, Heinz-Walter, Dr.
Bruesseler Ring 43
D-6700 Ludwigshafen (DE)**
Erfinder : **Taglieber, Volker, Dr.
Franz-Liszt-Strasse 15
D-6904 Eppelheim (DE)**
Erfinder : **Weiss, Franz-Josef, Dr.
Schilfweg 1
D-6708 Neuhofen (DE)**

## Beschreibung

Die Carbonylierung von Olefinen, d. h. die Umsetzung von beispielsweise Ethylen, Propylen oder Butylen mit Kohlenmonoxid und Alkanolen in Gegenwart von Carbonylkomplexen von Metallen der VIII. Gruppe des periodischen Systems, wird in der Technik in großem Maßstab zur Herstellung von Carbonsäureestern benutzt. Verwendet man als Ausgangsstoffe Diolefine, z. B. 1,3-Butadien, so erhält man über die Zwischenstufe Pentensäureester Adipinsäuredimethylester, ein wertvolles Ausgangsprodukt zur Herstellung von Faserrohstoffen. Da Kohlenmonoxid häufig in geringen Mengen Wasserstoff enthält oder durch Reaktion mit eingeschlepptem Wasser sich Wasserstoff bildet, tritt neben der Carbonylierungsreaktion auch eine Hydroformylierungsreaktion ein. Diese führt zu Aldehyden und durch Umsetzung mit vorhandenen Alkanolen zu Acetalen. Desweiteren treten synthesebedingt ungesättigte Ketone, z. B. Tridecenone und Butendicarbonsäureester als Nebenprodukte in niedrigen Konzentrationen auf. Sobald die Siedepunkte der Acetale, Aldehyde oder ungesättigten Ketone sowie Butendicarbonsäureester mit den erzeugten Estern sehr eng beieinander liegen, ist eine destillative Abtrennung dieser unerwünschten Nebenprodukte technisch außerordentlich aufwendig. Die Abtrennung von Aldehyden, Acetalen und ungesättigten Verbindungen hat besondere Bedeutung bei der Herstellung von Adipinsäureestern, da die daraus hergestellte Adipinsäure dann für die Herstellung von Polymeren mit Faserqualität wenig geeignet ist. Darüberhinaus führen selbst geringe Mengen an Aldehyden, Acetalen und ungesättigten Verbindungen zu Verfärbungen der erzeugten Produkte, die unerwünscht sind.

Es war deshalb die technische Aufgabe gestellt, ein Verfahren zur Verfügung zu stellen, das es ermöglicht, Acetale, Aldehyde und/oder ungesättigte Verbindungen aus Carbonsäureestern auf einfache Weise zu entfernen.

Diese Aufgabe wird gelöst in einem Verfahren zur Reinigung von Aldehyde, Acetate und/oder ungesättigte Verbindungen enthaltenden Carbonsäureestern, die durch Umsetzung von olefinisch ungesättigten Vebindungen mit Kohlenmonoxid und Alkanolen erhalten worden sind, wobei man die Aldehyde, Acetale und/oder ungesättigte Verbindungen enthaltenden Carbonsäureester mit Wasserstoff in Gegenwart von mit mindestens einem Metall der VIII. Gruppe des periodischen Systems dotierten sauren Ionenaustauschern oder Zeolithen bei erhöhter Temperatur behandelt und die gebildeten Leicht- und/oder Höhersieder destillativ abtrennt.

Das neue Verfahren hat den Vorteil, daß die Ester auf einfache Weise von den verunreinigenden Aldehyden, Acetalen und/oder ungesättigten Verbindungen befreit werden, die sonst unter erheblichem Aufwand mittels mehrstufiger Reinigungsverfahren abgetrennt werden müssen.

Die nach dem erfindungsgemäßen Verfahren gereinigten Carbonsäureester zeichnen sich durch sehr hohe Reinheit aus, da die Entfernung der Acetale, der Aldehyde und der ungesättigten Verbindungen quantitativ erfolgt bzw. der enthaltene Butendicarbonsäureester sogar zu Wertprodukt gewandelt wird.

Der Erfindung liegt der allgemeine Erfindungsgedanke zugrunde, die nahe bei den Carbonsäureestern siedenden Acetale katalytisch in Vinylether und durch anschließende Hydrierung in leichtsiedende Ester überzuführen und als solche abzutrennen oder die Aldehyde und Acetale durch Aldolisierung in Hochsieder zu überführen und als solche abzutrennen. Butendicarbonsäureester werden durch hydrierende Behandlung in Wertprodukte, d. h. Adipinsäureester, überführt. Die ungesättigten Ketone werden in gesättigte, destillativ abtrennbare Ketone umgewandelt.

Bevorzugte Carbonsäureester erhält man durch Carbonylierung von $C_2$- bis $C_{12}$-Monoolefinen, $C_4$- bis $C_{12}$-Diolefinen, $C_5$- bis $C_{12}$-Cycloalkenen oder $C_3$- bis $C_{12}$-Alkenmonocarbonsäure-$C_1$- bis $C_8$-Alkylestern. Die Carbonylierung erfolgt nach bekannten Verfahren durch Umsetzen mit Kohlenmonoxid und $C_1$- bis $C_8$-Alkanolen, insbesondere $C_1$- bis $C_4$-Alkanolen, z. B. bei Temperaturen von 100 bis 200 °C, und unter Drücken von 50 bis 1 000 bar in Gegenwart von Carbonylkomplexen von Metallen der VIII. Gruppe des Periodensystems, insbesondere von Kobalt- oder Rhodiumcarbonylkomplexen. Hieraus resultieren $C_3$- bis $C_{13}$-Monocarbonsäureester von Alkanolen mit 1 bis 8 Kohlenstoffatomen, $C_6$- bis $C_{14}$-Dicarbonsäureester von Alkanolen mit 1 bis 8 Kohlenstoffatomen oder Cycloalkancarbonsäureester mit 5 bis 12 Kohlenstoffatomen im Ring. Besonders bevorzugt sind gesättigte Mono- und Dicarbonsäureester mit den obengenannten Kohlenstoffzahlen. So hergestellte Ester enthalten als Nebenprodukte Aldehyde und Acetale, wobei der Aldehydteil die gleiche Kohlenstoffzahl aufweist wie die entsprechenden Carbonsäuren. Die Acetale enthalten darüberhinaus noch die jeweiligen Reste, die den mitverwendeten Alkanolen entsprechen. Darüberhinaus sind noch ungesättigte Ketone oder ungesättigte Dicarbonsäureester je nach Art der verwendeten Ausgangsstoffe enthalten. Der Gehalt an Aldehyden und Acetalen beträgt beispielsweise 0,1 bis 15 Gew.%. Geeignete Verfahren werden z. B. beschrieben in der US-PS 3 176 28 und DE-OS 16 18 156.

Besondere technische Bedeutung haben Adipinsäure-$C_1$- bis $C_4$-Alkylester erlangt, die durch Carbonylierung von Butadien oder Pentensäure-$C_1$- bis $C_4$-Alkylester mit Kohlenmonoxid und $C_1$- bis $C_4$-Alkanolen hergestellt worden sind.

Ein typisches Gemisch enthält beispielsweise neben Adipinsäure 9 bis 14 Gew.% Methylglutarsäureester, 2 bis 5 Gew.% Ethylbernsteinsäureester, 0,1 bis 0,3 Gew.% 5-Formylvaleriansäureester, 0,2 bis 0,5 Gew.% 6,6-Dimethoxycapronsäureester, 0,03 bis 0,1 Gew.% Butendicarbonsäurester sowie 0,005 bis 0,02 Gew.% Tridecenone. Geeignete Verfahren werden z. B. beschrieben in der US-PS 2 801 263 und DE-PS 2 713 195.

Geeignete Katalysatoren sind saure Ionenaustauscher und Zeolithe die mit mindestens einem Metall der VIII. Gruppe des periodischen Systems dotiert sind. Geeignete Metalle sind beispielsweise Kobalt, Nickel, Palladium oder Platin. Besonders bevorzugt werden Palladium oder Platin, insbesondere Palladium. Vorteilhaft haben die sauren Ionenaustauscher und Zeolithe einen Metallgehalt von 0,5 bis 5 Gew.% an den genannten Metallen. Geeignete Zeolithe sind beispielsweise A-, X- oder Y-Zeolihe, sowie natürliche Zeolithe, wie Fanjasite und Nordenite. Besonders bevorzugt sind stark saure Ionenaustauscher (vernetztes Polystyrol mit Sulfonsäuregruppen).

Die Katalysatoren werden z. B wie folgt hergestellt :

Der saure Ionentauscher wird in Wasser aufgeschlämmt, eine verdünnte Palladiumnitratlösung mit dem vorausberechneten Gehalt an Palladium unter Rühren zugegeben, abfiltriert, gewaschen und isoliert. Bei Verwendung für diskontinuierliche Arbeitsweise wird der Katalysator separat mit Methanol bis zur Wasserfreiheit gewaschen ; bei Verwendung in kontinuierlich betriebenen Reaktoren erfolgt die Trocknung zweckmäßigerweise im Reaktor selbst.

An den sauren Zentren des Katalysators wird die mengenmäßig bedeutendste Verunreinigung das Acetal (beispielsweise 6,6-Dimethoxycapronsäuremethylester) zu dem entsprechenden Vinylether und Methanol gespalten und der Vinylether zu dem gesättigten Ether hydriert. Diese Ether haben einen erheblich niedrigeren Siedepunkt als die Acetale und sind damit destillativ abtrennbar. Die mengenmäßig ebenfalls bedeutende Verunreinigung Aldehyd (z. B 5-Formylvaleriansäuremethylester) aldolisiert an den sauren Zentren zu dem entsprechenden Aldol, das aufgrund seines hohen Molekulargewichts einen wesentlich höheren Siedepunkt als der Aldehyd oder der Carbonsäureester hat. Die anderen störenden Verunreinigungen, wie ein- oder mehrfach ungesättigte Verbindungen, werden entweder in Wertprodukt oder in destillativ abtrennbare Verbindungen umgewandelt. Im Falle des Butendicarbonsäureresters führt die hydrierende Reinigung zum Adipinsäuredimethylester. Die ungesättigten Ketone werden zu gesättigten Ketonen, die leicht destillativ abtrennbar sind, umgewandelt. Das Verfahren wird bei erhöhter Temperatur durchgeführt. Bevorzugt wendet man Temperaturen von 50 bis 300 °C, insbesondere 100 bis 150 °C an. Falls man mit den gennanten Metallen dotierte saure Ionenaustauscher verwendet, hat es sich besonders bewährt, Temperaturen von 80 bis 140 °C einzuhalten.

Die Hydrierung mit Wasserstoff läßt sich bei Atmosphärendruck durchführen. Aus Gründen der erhöhten Wasserstofflöslichkeit hat es sich als günstig erwiesen, erhöhten Druck, z. B. bis zu 10 bar, einzuhalten. In der sich anschließenden Destillation brauchen die Carbonsäureester lediglich von wesentlich höher- bzw. niedrigersiedenden Komponenten abgetrennt werden. Es sei besonders hervorgehoben, daß diese Destillation keine besonders hohen Anforderungen im Bezug auf die Trennstufenzahl stellt.

Der Vorteil des Verfahrens liegt zum einen darin begründet, daß Carbonsäureester auf einfache Weise von mehreren Verunreinigungsarten gleichzeitig befreit und hochrein erhalten werden, so daß ansonsten notwendige, höchst aufwendige Trennverfahren entfallen können. Diese wären zur Sicherstellung der Faserqualität hieraus herstellbarer Dicarbonsäuren unabdingbar erforderlich. Insofern wird der technische Aufwand für die Herstellung reiner Carbonsäureester durch das erfindungsgemäße Reinigungsverfahren stark vermindert.

Zweitens stellt die erfindungsgemäße Verwendung eines Katalysators mit sauren Zentren, wie Zeolithen bzw. stark sauren Ionenaustauschern, die mit mindestens einem Metall der VIII. Gruppe des periodischen Systems dotiert sind, sicher, daß nur unerwünschte Verbindungen angegriffen werden, nicht jedoch die Esterfunktionen des Wertprodukts. Die Durchführung einer technisch üblichen Hydrierung mit üblichen Katalysatoren ohne die beschriebene Doppelfunktion würde hingegen auch unter milden Bedingungen durch Hydrierung der Esterfunktion zu Esteralkoholen (z. B. 6-Hydroxycapronsäuremethylester) führen, die schon bei geringer thermischer Beanspruchung unter Methanolabspaltung zu Lactonen, z. B. ε-Caprolacton, cyclisieren. Solche Lactone sind häufig vom Wertprodukt destillativ nicht mehr abzutrennen. Dies gilt insbesondere für die Herstellung von Adipinsäuredimethylsäureester, der dann für den Faserbereich ungeeignet wäre. Darüberhinaus müßte eine partielle Hydrierung von Esterfunktionen als realer Verlust an Wertprodukt kalkuliert werden. Hingegen stellt das erfindungsgemäße Reinigungsverfahren eine elegante, technisch leicht durchführbare Methode dar, Carbonsäureester zu reinigen, bei der sogar geringe Mengen an Wertprodukt hinzugewonnen werden.

Die erfindungsgemäß gereinigten Ester eignen sich als Lösungsmittel. Adipinsäureester werden zu Adipinsäure verseift, das ein Ausgangsprodukt zur Herstellung von Polykondensaten mit Polyamid-6,6 ist.

Das Verfahren nach der Erfindung sei an folgenden Beispielen veranschaulicht.

Beispiel 1

In einen 2-l-Schüttelautoklaven werden 0,2 l eines mit 1,0 Gew.% Palladium dotierten, stark sauren Ionenaustauschers (vernetztes Polystyrol mit Sulfonsäuregruppen) und 0,8 kg Adipinsäuredimethylester eingefüllt, der 0,3 Gew.% 6,6-Dimethoxycapronsäuremethylester, 0,25 Gew.% 5-Formylvaleriansäuremethylester, 0,07 Gew.% Butendicarbonsäuredimethylester, 0,015 Gew.% Tridecenone sowie 0,2 Gew.% 2-Methylgutarsäuredimethylester enthält und ein UV-Zahl von 20 260 aufweist. Es wird Wasserstoff von 5 bar Druck aufgepreßt und dann 2,5 Stunden auf 125 °C unter gleichzeitigem Schütteln erhitzt. Der nach dem Erkalten erhaltene Reaktionsaustrag weist eine UV-Zahl von 2 800 auf. Nach Rektifikation wird 99,75 gew.%iger Adipinsäuredimethylester der UV-Zahl 1 900 erhalten, der noch 0,1 Gew.% 2-Methylgutarsäuredimethylester enthält.

Beispiel 2

In einen mit 40 l stark saurem Ionenaustauscher, der 1,5 g Palladium pro l trockenem Ionenaustauscher enthält, befüllten 9 m Rohrreaktor werden stündlich 18 l 99,2 gew.%iger Adipinsäuredimethylester zusammen mit 80 Nl Wasserstoff eingebracht. Der Reaktor wird geflutet betrieben und bei einer Temperatur von 110 °C und einem Druck von 8 bar gehalten. Der eingesetzte Ester enthält durchschnittlich 0,5 Gew.% 6,6-Dimethoxycapronsäuremethylester, 0,13 Gew.% 5-Formylvaleriansäuremethylester, 0,02 Gew.% Butendicarbonsäuredimethylester, 0,02 Gew.% Tridecenone und Tetradecenone sowie 0,05 Gew.% 2-Methylgutarsäuredimethylester und weist eine UV-Zahl von durchschnittlich 14 000 auf. Der Reaktionsaustrag läuft einer Destillationskolonne mit 30 theoretischen Böden zu, aus der das Wertprodukt im Seitenstrom abgezogen wird. Der erhaltene Adipinsäuredimethylester hat einen Gehalt von 99,85 % und hat durchschnittlich eine UV-Zahl von 1 200.

**Ansprüche**

1. Verfahren zur Reinigung von Aldehyde, Acetale und/oder ungesättigte Verbindungen enthaltenden Carbonsäureestern, die durch Umsetzen von olefinisch ungesättigten Verbindungen mit Kohlenmonoxid und Alkanolen erhalten worden sind, dadurch gekennzeichnet, daß man die Aldehyde, Acetale und/oder ungesättigte Verbindungen enthaltenden Carbonsäureester mit Wasserstoff in Gegenwart von mit mindestens einem Metall der VIII. Gruppe des periodischen Systems dotierten sauren Ionenaustauschern oder Zeolithen bei erhöhter Temperatur behandelt und die gebildeten Leicht- und/oder Höhersieder destillativ abtrennt.

2. Verfahren nach Anspruch 1, dadurch gekennzeichnet, daß die sauren Ionenaustauscher oder Zeolithe mit Palladium oder Platin dotiert sind.

3. Verfahren nach den Ansprüchen 1 und 2, dadurch gekennzeichnet, daß man eine Temperatur von 80 bis 140 °C anwendet.

4. Verfahren nach den Ansprüchen 1 bis 3, dadurch gekennzeichnet, daß man stark saure Ionenaustauscher verwendet.

5. Verfahren nach den Ansprüchen 1 bis 4, dadurch gekennzeichnet, daß man Adipinsäuredimethylester, der mit 5-Formylvaleriansäuremethylester und/oder 6,6-Dimethoxycapronsäuremethylester und/oder Butendicarbonsäuredimethylester und/oder Tridecenonen verunreinigt ist, als Ausgangsgemisch verwendet.

**Claims**

1. A process for purifying a carboxylate which contains aldehydes, acetals and/or unsaturated compounds and is obtained by reacting an olefinically unsaturated compound with carbon monoxide and an alkanol, wherein the carboxylate containing aldehydes, acetals and/or unsaturated compounds is treated with hydrogen at elevated temperature in the presence of an acidic in exchanger or zeolite doped with one or more metals of group VIII of the periodic table, and the resulting low and/or high boilers are separated off by distillation.

2. A process as claimed in claim 1, wherein the acidic ion exchanger or the zeolite is doped with palladium or platinum.

3. A process as claimed in claims 1 and 2, wherein the temperature employed is from 80 to 140 °C.

4. A process as claimed in claims 1 to 3, wherein a strongly acidic ion exchanger is used.

5. A process as claimed in claims 1 to 4, wherein the starting mixture used is dimethyl adipate containing, as impurities, methyl 5-formylvalerate and/or methyl 6,6-dimethoxycaproate and/or dimethyl butenedicarboxylate and/or tridecenones.

**Revendications**

1. Procédé de purification d'esters d'acides carboxyliques, contenant des aldéhydes, des acétals et/ou des composés insaturés, esters qui ont été obtenus par réaction de composés à insaturation oléfinique avec de l'oxyde de carbone et des alcanols, caractérisé par le fait qu'on traite les esters d'acide carboxyliques, contenant des aldéhydes, acétals et/ou composés insaturés, avec de l'hydrogène, à température assez élevée, en présence d'échangeurs d'ions acides ou de zéolites, dopés avec au moins un métal du 8e groupe du tableau périodique ; et on sépare par distillation les produits formés à point d'ébullition faible et/ou plus élevé.

2. Procédé selon la revendication 1, caractérisé par le fait que les échangeurs d'ions acides ou les zéolites sont dopés avec du palladium ou du platine.

3. Procédé selon les revendications 1 ou 2 caractérisé par le fait que l'on applique une température de 80 à 140 °C.

4. Procédé selon les revendications 1 à 3, caractérisé par le fait que l'on utilise des échangeurs d'ions fortement acides.

5. Procédé selon les revendications 1 à 4, caractérisé par le fait que l'on utilise, comme mélange de départ, de l'ester diméthylique d'acide adipique qui est souillé d'ester méthylique d'acide 5-formylvalerianique et/ou d'ester méthylique d'acide 6,6-diméthoxy-capronique et/ou d'ester diméthylique d'acide butènecar-boxylique et/oude tridécénones.